# EUROPÄISCHE PATENTSCHRIFT

(11) **EP 2 081 541 B1**
(45) Veröffentlichungstag und Bekanntmachung des Hinweises auf die Patenterteilung: **04.07.2012**
(21) Anmeldenummer: 07821712.2
(22) Anmeldetag: 23.10.2007
(51) Int. Cl.: A61K 6/083

(54) **MIKROVERKAPSELTE PHOTOINITIATOREN UND DEREN VERWENDUNG FÜR DENTALMATERIALIEN**
MICROENCAPSULATED PHOTO-INITIATORS AND THE USE THEREOF FOR DENTAL MATERIALS
PHOTO-INITIATEURS MICRO-ENCAPSULÉS, ET LEUR UTILISATION POUR DES MATÉRIAUX DENTAIRES

(30) Priorität: 25.10.2006 DE 102006050153
(43) Veröffentlichungstag der Anmeldung: 29.07.2009
(73) Patentinhaber: Ivoclar Vivadent AG, 9494 Schaan (LI)
(72) Erfinder: SALZ, Ulrich, 88131 Lindau (DE); ZIMMERMANN, Jörg, 8046 Zürich (CH); RHEINBERGER, Volker, 9490 Vaduz (LI); LANDFESTER, Katharina, 89075 Ulm (DE); ZIENER, Ulrich, 89075 Ulm (DE); VOLZ, Marissa, 89075 Ulm (DE)
(74) Vertreter: Fitzner, Uwe
(86) Internationale Anmeldenummer: PCT/EP2007/061348
(87) Internationale Veröffentlichungsnummer: WO 2008/049839

(56) Entgegenhaltungen:
- US-A- 5 154 762
- DATABASE WPI Week 200577 Derwent Publications Ltd., London, GB; AN 2005-752905 XP002465323 & JP 2005 289961 A (MATSUKAZE KK) 20. Oktober 2005 (2005-10-20)

## Beschreibung

Die vorliegende Erfindung betrifft die Herstellung von mikroverkapselten Photoinitiatoren und deren Verwendung in Dentalmaterialien, insbesondere zur Verwendung in Adhäsiven, Beschichtungsmaterialien oder Zementen, sowie ein Verfahren zur Herstellung von selbstätzenden, selbstkonditionierenden Dentalmassen.

### Stand der Technik

Radikalisch polymerisierbare Dentalmaterialien, wie z.B. Versiegler, Dentin/Schmelz-Adhäsive, Befestigungsmaterialien oder Füllungskomposite werden in der Regel durch Bestrahlung mit Licht zur Aushärtung gebracht. Grund dafür ist die einfache Handhabung der lichthärtenden Materialien. Meist sind sie einkomponentig, d.h. sie müssen vor der Anwendung nicht angemischt werden, sie haben eine lange Verarbeitungszeit und härten dann, wenn gewünscht, bei Bestrahlung schnell aus. Zudem weisen sie auch eine gute Lagerstabilität bei Raumtemperatur auf. Wegen der Gewebeverträglichkeit und der ausreichenden Durchhärtung bei pigmentierten Systemen, wird heutzutage nahezu ausschließlich mit Licht im Wellenlängenbereich von 400 bis 500 nm bestrahlt. Eines der ersten in radikalisch polymerisierbaren Dentalmaterialien eingesetzten Photoinitiatorsysteme war die Kombination aus einem alpha-Diketon und einem Amincoinitiator (GB 1408265). Entsprechende Dentalzusammensetzungen, in denen dieses Photoinitiatorsystem eingesetzt wurde, sind z.B. in der US 4.457.818 oder US 4.525.256 beansprucht, wobei vorzugsweise Campherchinon als -Diketon eingesetzt wurde. Auch sind Diketonkombinationen wie z.B. die Kombination aus 1-Aryl-2-alkyl-1,2-ethandionen mit cyclischen Diketonen beschrieben worden (US 6.204.302). Photoinitiatorsysteme, bestehend nur aus einem Initiatormolekül, sog. -Spaltern wie Titanocene, Acylphosphonate, Acylphosphinoxide oder Bisacylphosphinoxide, finden in lichthärtenden Dentalmaterialen ebenfalls Einsatz. Allein sind Titanocene jedoch nicht besonders reaktiv und werden deshalb vorzugsweise in Kombination mit Aminen und/oder Peroxiden angewendet (EP 0334338). Acylphosphonate wie z.B. das Benzoyl-di(2,6-dimethylphenyl)phosphonat werden ebenfalls wegen ihrer geringen Durchhärtungstiefe bzw. -reaktivität bevorzugt in Kombination mit einem zweiten Initiatorsystem, wie z.B. dem Campherchinon/Aminsystem, angewendet (EP 0336417). Acylphosphinoxide, wie z.B. das in DE 2909992 beschriebene 2,4,6-Trimethylbenzoyl-diphenylphosphinoxid, werden ebenfalls bevorzugt in Dentalkomposite eingesetzt (EP 0173567). In EP 1236459 wird ebenfalls ein dentales Komposit beschrieben, das mittels Acylphosphinoxid lichtgehärtet werden kann, mit dem Unterschied zur EP 0173567, dass eine spezielle Mischung aus Füllstoffpartikeln Verwendung findet. EP 0948955 beschreibt eine dentale Zusammensetzung, bei der eine Initiatorkombination aus Acylphosphinoxid, organischem Peroxid, tertiärem Amin und aromatischer Sulfinsäure bzw. einem Salz davon zum Einsatz kommt. Eine antibakterielle Adhäsivzusammensetzung wird in US 2002/0035169 beansprucht, bei der als Initiatorsystem bevorzugt eine Mischung aus Acylphosphinoxid und einem alpha-Diketon eingesetzt wird.

Bisacylphosphinoxide und deren Anwendung als Initiatoren für die Photopolymerisation von Verbindungen mit C-C-Doppelbindungen wurden erstmals in der DE 3443221 beschrieben. Die DE 3801511 beschreibt photopolymerisierbare Dentalmassen, die in zwei Schritten ausgehärtet werden können. Die darin beschriebenen Massen enthalten unter anderem eine Photoinitiatorkomponente I mit einem Absorptionsmaximum von < 450 nm sowie eine Photoinitiatorkomponente II mit einem Absorptionsmaximum von > 450 nm, wobei als Photoinitiatorkomponente I ein Bisacylphosphinoxid und als Photoinitiatorkomponente II ein -Diketon Anwendung finden. In der DE 3837569 wird ebenfalls eine photopolymerisierbare Dentalmasse, die ein Bisacylphosphinoxid als Photoinitiator enthält, beansprucht, wobei damit eine Thiol-En-Polymerisation initiiert wird. Weiterhin sind Bisacylphosphinoxide auch in der US 5.399.770 (Alkylbisacylphosphinoxide), DE 19532358 (Alkoxyphenylsubstituierte Bisacylphosphinoxide) oder WO 03/019295 (Bathochrome Mono- und Bisacylphosphinoxide), beschrieben worden.

Nachteilig bei Photoinitiatorsystemen, die aus einem alpha-Diketon und einem Amin aufgebaut sind, ist, dass sie für den Einsatz in selbstätzenden, selbstkonditionierenden dentalen Restaurationsmaterialien nur bedingt tauglich sind. Selbstätzende, setbstkonditionierende Dentalmaterialien zeichnen sich dadurch aus, dass keine Prekonditionierung der Zahnhartsubstanz notwendig ist. Dazu zählen die selbstätzenden Dentin/Schmelzadhäsive, selbstätzende Beschichtungsmaterialien, Methacrylat-verstärkte Glasionomerzemente sowie selbsthaftende Komposite oder auch so genannte Compomere. Sie sind meist derart aufgebaut, dass sie ein oder mehrere Haftmonomere mit Säurefunktion, ein oder mehrere nicht saure Comonomere und ein Photoinitiatorsystem sowie weitere Additive wie Füllstoffe oder gegebenenfalls auch Lösungsmittel enthalten. Werden in diesen Systemen alpha-Diketon/Amin-Photoinitiatorsysteme wie oben beschrieben eingesetzt, besteht das Problem, dass Amine unter sauren Bedingungen protoniert und abgebaut werden und dadurch das Initiatorsystem an Effizienz verliert.

Photoinitiatoren, wie die Acyl- bzw. Bisacylphosphinoxide, die durch monomolekulare Bindungsspaltung, sog. Norrish-Typ 1-Spaltung, polymerisationsauslösende Radikale bilden, zeigen den Nachteil, dass die in beiden Molekülen vorkommende Kohlenstoff-Phosphor-Bindung leicht durch nucleophile Verbindungen, wie z.B. Wasser oder Alkohole gespalten wird (Crivello J.V. and Dietliker K.: "Photoinitiators for free radical cationic & anionic photopolymerization", in: Surface Coatings Technology, Bradley G (ed.), 2nd Edition, John Wiley & Sons, 1998). Dadurch wird der Photoinitiator sukzessive abgebaut, die Initiierung des entsprechenden Restaurationsmaterials ist nur noch unzureichend, es härtet unvollständig aus. Dies bedeutet, dass derart aufgebaute Dentalmaterialien ebenfalls bei Lagerung ihre mechanischen Eigenschaften verlieren und das entsprechende Adhäsiv, das Beschichtungsmaterial oder der Befestigungszement mit der Zeit seine klinische Tauglichkeit verliert. Die Mikroverkapselung von Initiatoren zur Anwendung in Dentalmaterialien wurde z.B. in US 5.154.762 sowie WO 03/057792 bereits beschrieben. Dabei wurden jedoch keine Photoinitiatoren mikroverkapselt, sondern nur Redoxinitiatoranteile von chemisch härtenden Dentalmaterialien, bestehend aus mehreren Komponenten, mit dem Ziel, diese in eine Komponente bringen zu können, ohne dass diese gleich miteinander reagieren. Bei der Anwendung muss die Kapsel mechanisch zerstört werden, um das Redoxinitiatorsystem zu aktivieren.

Die JP 2004330704 A erwähnt zwar Mikrokapseln mit Acylphosphinoxiden. Doch handelt es sich nicht um den Bereich der Dentaltechnik.

Die DE 19906834 A1 betrifft dentale Glasionomerzementmassen vom Pastentyp. Diese umfassen eine erste Paste, die ein alpha-, beta-ungesättigtes Carbonsäurepolymer, Wasser und einen mit den alpha-, beta-ungesättigten Carbonsäurepolymer nicht-reaktiven Füllstoff umfasst, sowie eine zweite Paste, die ein Fluoraluminosilicatglaspulver und ein säuregruppenfreies polymerisierbares Monomer umfasst. Eine der beiden Pasten enthält einen Polymerisationskatalysator. Um ein Härten der Masse zu erreichen, werden beide Pasten miteinander vermischt. Eine Miniemulsion wird aber nicht erwähnt.

Aus der DE 60116142 T2 sind Dentalzusammensetzungen bekannt. Es handelt sich um ein Zweikomponentensystem aus den Teilen A und B. Demnach befinden sich die Initiatoren abwechselnd in A oder B. Hier werden Radikalinitiatoren, z. B. Acylphosphinoxide eingesetzt. Ferner wird der Einsatz von Peroxiden und Aminen erwähnt. Diese können mikroverkapselt sein.

Aus dieser Entgegenhaltung gehen Einkomponentensysteme nicht hervor.

Die DE 102004020726A1 betrifft ein Verfahren zur Herstellung einer wässrigen Dispersion polymerverkapselter Pigmente. Es handelt sich also nicht um die Verkapselung von Initiatoren.

Aus JP 2005 289961 A ist es bekannt, Mikrokapseln herzustellen, die aus beliebigen Hüllmaterialien und Kernmaterialien bestehen. Diese Mikrokapseln müssen durch äußeren Einfluss zerstört werden, damit die Kernmaterialien zugänglich werden.

### Aufgabe der Erfindung

Der Erfindung liegt die Aufgabe zugrunde, Photoinitiatorsysteme bereitzustellen, die im sichtbarem Bereich polymerisationsauslösend sind und in Gegenwart von Wasser, Alkoholen und Säuren eine verbesserte Lagerstabilität zeigen und damit für selbstätzende, selbstkonditionierende Dentalmassen, vor allem zur Herstellung von selbstätzenden Dentin/Schmelzadhäsiven, selbstätzenden Beschichtungsmaterialien sowie Methacrylat-verstärkten Glasionomerzementen für dentale Zwecke besonders geeignet sind, wobei eine Aktivierung des Initiatorsystems durch mechanische Zerstörung der Kapseln nicht notwendig ist.

### Lösung der Aufgabe der Erfindung

Die Aufgabe wird durch Mikrokapseln bestehend aus einer Hülle aus Polymeren und aus einem Kern enthaltend Photoinitiatoren, welche Acylphosphinoxide, Bisacylphosphinoxide, Derivate davon oder Amincoinitiatoren oder Gemische dieser Verbindungen aufweisen, wobei
a) die Photoinitiatoren Acylphosphinoxide der allgemeinen Formel (I) 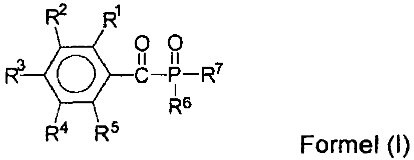 enthalten wobei
   R¹, R², R³, R⁴ und R⁵ unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy substituiertes Phenyl sowie R⁶ und R⁷ 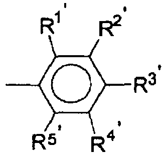 sein kann und R⁶ und R⁷ unabhängig voneinander unterschiedliche Substituenten aufweisen können, wobei
   R^{1'}, R^{2'}, R^{3'}, R^{4'} und R^{5'} unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxysubstituiertes Phenyl sein kann,
   oder
b) die Photoinitiatoren Bisacylphosphinoxide der allgemeinen Formel (II) 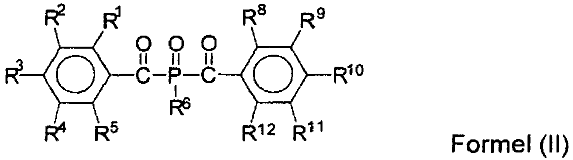 enthalten, wobei
   R¹, R², R³, R⁴, R⁵, R⁸, R⁹, R¹⁰, R¹¹ und R¹² unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy-substituiertes Phenyl sein kann, sowie R⁶ 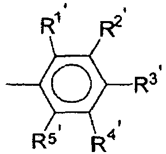 sein kann,
   wobei R^{1'}, R^{2'}, R^{3'}, R^{4'}, und R^{5'} unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy substituiertes Phenyl sein kann,
   oder
c) die Photoinitiatoren Amincoinitiatoren der allgemeinen Formel (III) 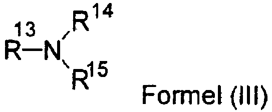 enthalten, wobei
   R¹³ C₁-C₂₀-Alkyl, durch ein oder mehrere O-Atome unterbrochenes C₂-C₁₈-Alkyl, durch CN, OH substituiertes C₁-C₄-Alkyl sein kann,
   R¹⁴ und R¹⁵ unabhängig voneinander C₁-C₂₀-Alkyl, durch ein oder mehrere O-Atome unterbrochenes C₂-C₁₈-Alkyl, durch CN, OH substituiertes C₁-C₄-Alkyl, unsubstituiertes oder mit ein, zwei oder drei C₁-C₆-Alkyl oder/und mit carboxyalkyl-substituiertes Phenyl sein kann,
   und wobei
d) die Hülle als Polymere Polymethacrylate oder Polymethacrylamide oder Mischungen daraus eingesetzt werden, gelöst. Die Problematik des Abbaus der Initiatoren wird demgemäß erfindungsgemäß derart gelöst, dass die Photoinitiatoren durch Mikroverkapselung in einer Polymerhülle geschützt werden und nur ein bestimmter Anteil nach und nach freigesetzt wird. Dabei handelt es sich um einen Depoteffekt, bei dem der Anteil aus den Kapseln nachgeliefert wird, der sich zersetzt hat, so dass ein konstantes Niveau an Photoinitiator in der kontinuierlichen Phase erreicht wird.

### Beschreibung der Erfindung

Erfindungsgemäß eingesetzt werden Acylphosphinoxide der allgemeinen Formel (I) 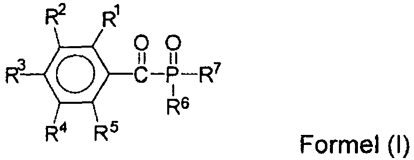 wobei
R¹, R², R³, R⁴ und R⁵ unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy substituiertes Phenyl sein kann sowie R⁶ und R⁷ 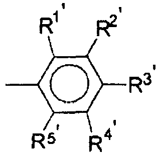 sind und für R⁶ und R⁷ unabhängig voneinander unterschiedliche Substituenten aufweisen können, wobei
R^{1'}, R^{2'}, R^{3'}, R^{4'}und R^{5'} unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy substituiertes Phenyl sein kann

Erfindungsgemäß eingesetzt werden alternativ Bisacylphosphinoxide der allgemeinen Formel (II), 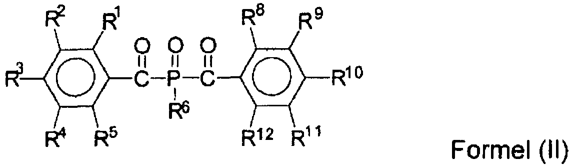 wobei
R¹, R², R³, R⁴, R⁵, R⁸, R⁹, R¹⁰, R¹¹ und R¹² unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy substituiertes Phenyl sein kann sowieR⁶ 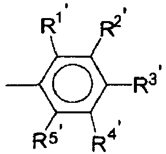 sein kann, wobei
R^{1'}, R^{2'}, R^{3'}, R^{4'} und R^{5'} unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy substituiertes Phenyl sein kann.

Acylphosphinoxide sowie Bisacylphosphinoxide sind kommerziell erhältlich. So wird das Acylphosphinoxid 2,4,6-Trimethyl-benzoyl-diphenylphosphinoxid zum Beispiel unter dem Handelsnamen Darocur® TPO sowie Lucirin® TPO kommerziell vertrieben, das Bisacylphosphinoxid Bis(2, 4,6-Trimethylbenzoyl)phenylphosphinoxid ist zum Beispiel unter dem Handelsnamen Irgacure® 819 im Handel erhältlich.

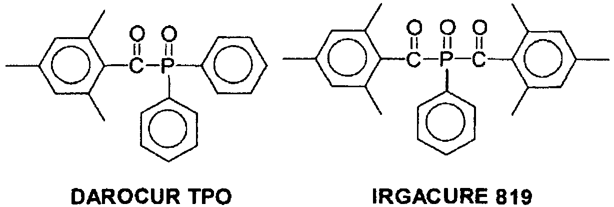

Darocur® und Irgacure® sind eingetragene Markenzeichen der Firma Ciba Specialities, Lucirin® ist ein eingetragenes Markenzeichen der Firma BASF AG.

Zudem sind Acylphosphinoxide sowie Bisacylphosphinoxide synthetisch relativ einfach zugänglich. So wird z.B. in DE 2909992 ausführlich die Synthese von Acylphosphinoxiden beschrieben. Dabei wird ein Säurehalogenid mit einem entsprechenden Phosphin umgesetzt. Im Fall des 2,4,6-Trimethylbenzoyldiphenylphosphinoxid wird 2,4,6-Trimethylbenzoylchlorid mit Methoxydiphenylphosphin umgesetzt. In DE 19708294 ist zum Beispiel ausführlich die Synthese von Bisacylphosphinoxiden beschrieben. So können Verbindungen der Formel (II) hergestellt werden durch doppelte Acylierung eines primären Phosphins mit mindestens 2 Äquivalenten eines Säurechlorids in Gegenwart einer Base und anschliessender Oxidation des erhaltenen Diacylphosphins zum entsprechenden Bisacylphosphinoxid. So wird im Fall des Bis-(2, 4,6-trimethylbenzoyl)phenylphosphinoxid zunächst Dichlorphenylphosphin mit elementarem Lithium umgesetzt. Danach bildet sich aus dem entstehenden Phenylphosphindilithium durch Zugabe von 2 Äquivalenten 2,4,6-Trimethylbenzoesäurechlorid das Bis-(2, 4,6-trimethylbenzoyl)phenylphosphin, welches mittels H₂O₂ anschliessend zum Bis-(2,4,6-trimethylbenzoyl)phenylphosphinoxid oxidiert wird.

Erfindungsgemäß eingesetzt werden alternativ außerdem Amincoinitiatoren der allgemeinen Formel (III), 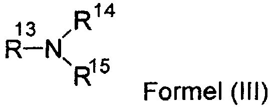 wobei
R¹³ C₁-C₂₀-Alkyl, durch ein oder mehrere O-Atome unterbrochenes C₂-C₁₈-Alkyl, durch CN, OH substituiertes C₁-C₄-Alkyl sein kann,
R¹⁴ und R¹⁵ unabhängig voneinander C₁-C₂₀-Alkyl, durch ein oder mehrere O-Atome unterbrochenes C₂-C₁₈-Alkyl, durch CN, OH substituiertes C₁-C₄-Alkyl, unsubstituiertes oder mit ein, zwei oder drei C₁-C₆-Alkyl oder/und mit carboxyalkyl substituiertes Phenyl sein kann.

Unter Amincoinitiatoren werden vorzugsweise trifunktionale Amine verstanden, welche unter Lichtwirkung eine schnelle Härtung begünstigen, sofern sie in Kombination mit einem Photosensitizer eingesetzt werden. Ein Beispiel für einen solchen sind Benzophenone.

Besonders bevorzugte Amincoinitiatoren der allgemeinen Formel (III) sind Ester der p-Dimethylaminobenzoesäure wie zum Beispiel Ethyl-4-dimethylaminobenzoat, 2-Ethylhexyl-4-dimethylaminobenzoat oder Benzophenonderivate wie das 4-(Dimethylamino)benzophenon oder Anilinderivate wie das 3,5,N,N-Tetramethylanilin, das 4,N,N-Trimethylanilin, das 4-tert.-Butyl-N,N-dimethylanilin, das N-Cyanoethyl-N-Methyl-Anilin oder das 2,4,6,N,N-Pentamethylanilin, aber auch nichtaromatische Amine wie das Triethanolamin oder N-Methyl-Diethanolamin.

Tertiäre Amincoinitiatoren sind kommerziell erhältlich, 2-Ethylhexyl-4-dimethylaminobenzoat wird z.B. unter dem Namen Genocure® EHA bzw. Ethyl-4-dimethylaminobenzoat unter dem Namen Genocure® EPD gehandelt (Rahn Chemie Zürich).

Unter "carboxyalkyl substituiertes Phenyl" sind Verbindungen der Formel IV 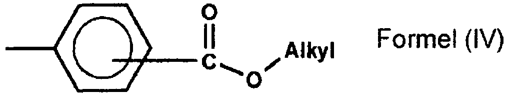 zu verstehen.

Diese kann als ein Substituent R¹⁴ und/oder R¹⁵ für die Amincoinitiatoren der Formel (III) dienen. Die Darstellung bedeutet, dass der Phenylrest am Stickstoff des tertiären Amins gebunden ist und die Carboxyalkyl-Gruppe beliebig am Phenylring gebunden sein kann. Die Zahl der C-Atome der Alkylgruppe liegt im Bereich C₁ bis C₈.

Als Polymere für die Hülle des Photonitiatorsystems werden erfindungsgemäß Polymethacrylate oder Polymethacrylamide oder Mischungen daraus eingesetzt, wobei durch Einsatz von bestimmten Mengen an mehrfach funktionalisierten (Meth)acrylverbindungen sich der Vernetzungsgrad des Polymeren und somit die Freisetzungsrate an Photoinitiator einstellen lässt.

Die Herstellung der erfindungsgemäßen Mikrokapseln erfolgt über den Weg einer Miniemulsion (N. Bechthold, F. Tiarks, M. Willert, K. Landfester, and M. Antonietti "Miniemulsion polymerization: Applications and new materials" Macromol. Symp., 2000, 151, 549-555), besonders bevorzugt über die direkte Miniemulsion (Öl in Wasser). Dazu wird zuerst ein Zweiphasensystem bestehend aus einer Mischung aus
i) dem zu verkapselnden Photoinitiator nach Formel (1), (2) oder (3), oder einer Mischung daraus,
ii) mindestens einem radikalisch polymerisierbaren Monomer,
iii) mindestens einer ultrahydrophoben Verbindung und
iv) mindestens einem Initiator zur thermischen Initiierung der radikalischen Polymerisation
   in eine Mischung aus Wasser und mindestens einem Tensid gegeben und
vi) anschließend mit Ultraschall versetzt um eine stabile Miniemulsion zu bilden.

Durch anschließende Erwärmung werden die gebildeten Monomertröpfchen polymerisiert, wodurch sich die erfindungsgemäßen Mikrokapseln bilden. Nach Aufreinigung und Trocknung lassen sich die auf diese Weise gewonnenen Mikrokapseln in dem erfindungsgemäßen Dentalmaterial redispergieren.

Die erfindungsgemäßen Miniemulsionen enthalten vorzugsweise bezogen auf das Gesamtgewicht der Miniemulsion:
- 0,1 bis 40 Gew.-% von dem zu verkapselnden Photoinitiator nach Formel (I), (II) oder (III), oder einer Mischung daraus, besonders bevorzugt 0,5 bis 20 Gew.%,
- 1,0 bis 80 Gew.-% polymerisierbares Monomer, besonders bevorzugt 2,0 bis 50 Gew.-%,
- zwischen 0,1 und 3 Gew.-%, besonders bevorzugt zwischen 0.5 und 2 Gew.-% der ultrahydrophoben Verbindung,
- 0,05 bis 5 Gew.-% von mindestens einem Initiator zur thermischen Initiierung der radikalischen Polymerisation, besonders bevorzugt 0,1 bis 3 Gew.-%,
- 0,01 bis 5 Gew.-% von mindestens einem Tensid, besonders bevorzugt 0,1 bis 3 Gew.-%,
wobei die Angaben der Gew.-% sich auf jeweils 100 Gew.-% addieren.

Als radikalisch polymerisierbare Monomere können mono- oder mehrfach funktionelle (Meth)acrylate bzw. (Meth)acrylamide ((Meth)acrylverbindungen) eingesetzt werden. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden. Mehrfach funktionelle Monomere haben vernetzende Eigenschaften.

Bevorzugte monofunktionelle (Meth)acrylverbindungen sind kommerziell verfügbare monofunktionelle Monomere, wie Methyl-, Ethyl-, Butyl-, Benzyl-, Furfuryl- oder Phenyl(meth)acrylat sowie 2-Hydroxyethyl- oder -propyl(meth)acrylat.

Bevorzugte mehrfach funktionelle (Meth)acrylverbindungen sind Bisphenol-A-di(meth)acrylat, Bis-GMA (ein Additionsprodukt aus Methacrylsäure und Bisphenol-A-diglycidylether), ethoxyliertes Bisphenol-A-di(meth)acrylat, UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Butandioldi-(meth)acrylat, 1,10-Decandioldi(meth)acrylat oder 1,12-Dodecandiol-di(meth)acrylat, vernetzende Pyrrolidone, wie z.B. 1,6-Bis-(3-vinyl-2-pyrrolidonyl)-hexan, oder kommerziell zugängliche Bisacrylamide wie Methylen- oder Ethylenbisacrylamid, Bis-(meth)acrylamide, wie z.B. N,N"-Diethyl-1,3-bis-(acrylamido)-propan, 1,3-Bis-(methacrylamido)-propan, 1,4-Bis-(acrylamido)-butan oder 1,4-Bis-(acryloyl)-piperazin, die durch Umsetzung aus den entsprechenden Diaminen mit (Meth)acrylsäurechlorid synthetisiert werden können.

Als ultrahydrophobe Verbindungen werden sehr unpolare organische Verbindungen wie insbesondere aliphatische oder aromatische Kohlenwasserstoffe eingesetzt. Unter den aliphatischen Kohlenwasserstoffen sind insbesondere die C₆-C₂₀-Alkane bevorzugt. Ganz besonders bevorzugt sind solche ausgewählt aus der Gruppe der aliphatischen Kohlenwasserstoffe, insbesondere bestehend aus Hexadecan.

Als Tensid werden für die Herstellung von Emulsionen bekannte ionische und nicht-ionische amphiphile Verbindungen eingesetzt. Bevorzugt wird das anionische Natriumdodecylsulfat, das kationische Cetyltrimethylammoniumchlorid, das nicht-ionische C₁₆-EO₅₀ (Lutensol® AT50) oder Blockcopolymere einsetzt.

Um sicherzustellen, dass die Polymerisation des relativ hydrophilen Monomers (z.B. Methylmethacrylat) nur in den Photoinitiator enthaltenden Tröpfchen abläuft und sich keine Sekundärpartikel bilden, wird als thermischer Initiator bevorzugt eine hydrophobe Diazoverbindung eingesetzt. Durch die thermische Initiierung wird das Monomer derart polymerisiert, dass sich eine Schale am Interface des Tröpfchens zur kontinuierlichen Phase bildet. Im Stadium der Miniemulsion sind das Monomer und der Photoinitiator nach Formel (1) bzw. (2) mischbar, während der Polymerisation tritt jedoch Phasenseparation auf. Durch den hydrophoben Charakter des Photoinitiators nach Formel (1) bzw. (2) und dem hydrophileren Charakter des Polymeren bilden sich Mikrokapseln mit einer Kern-Schale-Geometrie, wobei sich der Photoinitiator im Kern befindet, eingekapselt in einer Polymerschale.

Bevorzugte Diazoinitiatoren sind die kommerziell verfügbaren Verbindungen zum Starten von radikalische Polymerisationen wie z.B. AIBN oder 2,2'-Azobis-(2-methylbutyronitril).

Die beschriebenen Photoinitiatorsysteme eignen sich vor allen Dingen für die Verwendung für selbstätzende, selbstkonditionierende Dentalmassen. Besonders eignen sie sich für den Einsatz von Dentin/Schmelzadhäsiven. Derartige Dentalmaterialien eignen sich demgemäß bevorzugt als selbsthaftende Beschichtungsmaterialien und/oder selbstkonditionierende Befestigungszemente, besonders bevorzugt als selbstätzende Adhäsive.

Vorzugsweise können Dentalmaterialien, vorzugsweise in Form von selbstätzenden, selbstkonditionierenden Dentalmassen, in folgender Zusammensetzung mit den erfindungsgemäßen Mikrokapseln hergestellt werden:
a) 0,05 bis 20,0 Gew.-%, bevorzugt 0,5 bis 15 Gew.-% und besonders bevorzugt 0,5 bis 10 Gew.-% mikroverkapselten Photoinitiator der Formel (I) oder (II) oder (III) oder Mischungen daraus;
b) 0 bis 10,0 Gew.-%, bevorzugt 0,05 bis 7,5 Gew.-% und besonders bevorzugt 0,1 bis 5,0 Gew.-% -Diketon ;
c) 0 bis 10,0 Gew.-%, bevorzugt 0,1 bis 5 Gew.-% nicht mikroverkapselten Photoinitiator der Formel (I) oder (II) oder (III) oder Mischungen daraus;
d) 5 bis 95 Gew.-%, bevorzugt 5 bis 85 Gew.-% und besonders bevorzugt 5 bis 70 Gew.-% mono- oder multifunktionelles Monomer;
e) 0 bis 60 Gew.-%, bevorzugt 5 bis 50 Gew.-% und besonders bevorzugt 5 bis 45 Gew.-% acides radikalisch polymerisierbares Monomer;
f) 0 bis 80 Gew.-%, bevorzugt 5 bis 60 Gew.-% und besonders bevorzugt 10 bis 40 Gew.-% Lösungsmittel;
g) 0 bis 85 Gew.-% Füllstoff, besonders bevorzugt 1 bis 75 Gew.-% und
h) 0,01 - 5,0 Gew.-% Pigmente, Inhibitoren und Stabilisatoren,
wobei die Angaben der Gew.-% sich jeweils auf 100 Gew.-% addieren.

Dentalmaterialien, vorzugsweise in Form von selbstätzenden, selbstkonditionierenden Dentalmassen, zur Verwendung als Adhäsiv oder Beschichtungsmaterial enthalten vorzugsweise 1 bis 30 Gew.-% Füllstoff und Dentalmaterialien zur Verwendung als Zement 20 bis 85 Gew.-% Füllstoff. In Adhäsiven und Beschichtungsmaterialien werden zudem vorzugsweise 5 bis 60 Gew.-% Lösungsmittel eingesetzt. Bevorzugte Lösungsmittel sind Wasser, Methanol, Ethanol, Isopropanol, Ethylacetat, Aceton und Mischungen daraus.

Die erfindungsgemäß unter Verwendung der beschriebenen Photoinitiatoren erhaltenen Dentalwerkstoffe können als radikalisch polymerisierbare Monomere, mono- oder mehrfach funktionelle (Meth)acrylate bzw. (Meth)acrylamide ((Meth)acrylverbindungen) enthalten. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden. Mehrfach funktionelle Monomere haben vernetzende Eigenschaften.

Bevorzugte monofunktionelle (Meth)acrylverbindungen sind kommerziell verfügbare monofunktionelle Monomere, wie Methyl-, Ethyl-, Butyl-, Benzyl-, Furfuryl- oder Phenyl(meth)acrylat sowie 2-Hydroxyethyl- oder - propyl(meth)acrylat.

Besonders bevorzugt sind hydrolysestabile Monomere wie hydrolysestabile Mono(meth)acrylate, z.B. Mesitylmethacrylat oder 2-(Alkoxymethyl)acrylsäuren, z.B. 2-(Ethoxymethyl)acrylsäure, 2-(Hydroxymethyl)acrylsäure, N-mono- oder -disubstituierte Acrylamide, wie z.B. N-Ethylacrylamid, N,N-Dimethacrylamid, N-(2-Hydroxyethyl)acrylamid oder N-Methyl-N-(2-hydroxyethyl)acrylamid, und N-monosubstituierte Methacrylamide, wie z.B. N-Ethylmethacrylamid oder N-(2-Hydroxyethyl)methacrylamid sowie außerdem N-Vinylpyrrolidon und Allylether. Diese Monomere sind bei Raumtemperatur flüssig und eignen sich daher auch als Verdünner.

Bevorzugte mehrfach funktionelle Monomere sind Bisphenol-A-di(meth)acrylat, Bis-GMA (ein Additionsprodukt aus Methacrylsäure und Bisphenol-A-diglycidylether), ethoxyliertes Bisphenol-A-di(meth)acrylat, UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Butandioldi-(meth)acrylat, 1,10-Decandioldi(meth)acrylat oder 1,12-Dodecandiol-di(meth)acrylat.

Besonders bevorzugt sind hydrolysestabile Vernetzermonomere, wie z.B. vernetzende Pyrrolidone, wie z.B. 1,6-Bis-(3-vinyl-2-pyrrolidonyl)-hexan, oder kommerziell zugängliche Bisacrylamide wie Methylen- oder Ethylenbisacrylamid, Bis-(meth)acrylamide, wie z.B. N,N'-Diethyl-1,3-bis-(acrylamido)-propan (DBAP), 1,3-Bis-(methacrylamido)-propan, 1,4-Bis-(acrylamido)-butan oder 1,4-Bis-(acryloyl)-piperazin, die durch Umsetzung aus den entsprechenden Diaminen mit (Meth)acrylsäurechorid synthetisiert werden können.

Die erfindungsgemäß unter Verwendung der beschriebenen Photoinitiatoren erhaltenen Dentalmaterialien enthalten vorzugsweise auch mindestens ein radikalisch polymerisierbares, säuregruppenhaltiges Monomer. Bevorzugte Säuregruppen sind Carbonsäuregruppen, Phosphonsäuregruppen, Phosphatgruppen und/oder Sulfonsäuregruppen, wobei diese Gruppen in der Säureform, als Anhydrid oder in Form eines Esters vorliegen können. Besonders bevorzugt sind Monomere mit Phosphonsäuregruppen oder Phosphatgruppen. Die Monomere können eine oder mehrere acide Gruppen aufweisen, bevorzugt sind Verbindungen mit 1 bis 2 aciden Gruppen.

Bevorzugte polymerisationsfähige Carbonsäuren sind Maleinsäure, Acrylsäure, Methacrylsäure, 2-(Hydroxymethyl)acrylsäure, 4-(Meth)acryloyloxyethyltrimellitsäure bzw. das entsprechende Anhydrid, 10-Methacryloyloxydecylmalonsäure, N-(2-Hydroxy-3-methacryloyloxypropyl)-N-phenylglycin, N-Acryloyl-□-asparaginsäure (AAA) und 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure 2-[4-(Dihydroxyphosphoryl)-2-oxa-butyl]-acrylsäure, 2-[2-Dihydroxyphosphoryl)-ethoxy-methyl]-acrylsäure-2,4,6-trimethyl-phenylester und 2-[2-Dihydroxyphosphoryl)-ethoxy-methyl]-acrylsäure-ethylester (DPEAE).

Bevorzugte acide polymerisationsfähige Phosphorsäureester sind 2-Methacryloyloxypropylmono- und -dihydrogenphosphat, 2-Methacryloyloxyethylmono- und -dihydrogenphosphat, 2-Methacryloyloxyethyl-phenylhydrogenphosphat, Dipentaerythritol-pentamethacryloyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat, 1,3-Bis-(N-acryloyl-N-propyl-amino)-propan-2-yl-dihydrogenphosphat und 1,3-Bis-(methacrylamido)-propan-2-yl-dihydrogenphosphat (BM PP).

Bevorzugte polymerisationsfähige Sulfonsäuren sind Vinylsulfonsäure, 4-Vinylphenylsulfonsäure oder 3-(Methacrylamido)propylsulfonsäure.

Als mono- oder multifunktionelles Monomer können auch polymerisierbare Verbindungen zum Einsatz kommen, die eine antimikrobielle Wirkung aufweisen, wie z.B. 12-Methacryloyloxydodecylpyridiniumbromid, besonders bevorzugt sind so genannte Makromere, die einen polymeren Spacer zwischen der polymerisierbaren und der antimikrobiell wirksamen Gruppe enthalten.

Weiterhin können die Dentalmaterialien zur Verbesserung der mechanischen Eigenschaften oder zur Einstellung der Viskosität organische oder anorganische partikuläre Füllstoffe enthalten. Bevorzugte anorganische partikuläre Füllstoffe sind amorphe kugelförmige Materialien auf der Basis von Oxiden, wie ZrO₂ und TiO₂, nanopartikuläre oder mikrofeine Füllstoffe, wie pyrogene Kieselsäure nanopartikuläres Al₂O₃, Ta₂O₅, Yb₂O₃, ZrO₂, Ag oder TiO₂ bzw. Mischoxide aus SiO₂, ZrO₂ und/oder TiO₂ oder Fällungskieselsäure sowie Minifüllstoffe, wie Quarz-, Glaskeramik- oder Glaspulver mit einer durchschnittlichen Teilchengröße von 0,01 bis 5 m sowie röntgenopake Füllstoffe, wie Ytterbiumtrifluorid oder nanopartikuläres Bariumsulfat. Besonders geeignet sind Füllstoffe, die mit polymerisationsfähigen Gruppen oberflächenmodifiziert sind.

Außerdem können die mit den beschriebenen Photoinitiatoren hergestellten Dentalmaterialien ein oder mehrere weitere Additive enthalten, die aus Stabilisatoren, Aromastoffen, Farbstoffen, Pigmenten, Fluoridionen abgebenden Additiven, optischen Aufhellern, Weichmachern und/oder UV-Absorbern ausgewählt sind. Ein bevorzugter UV-Absorber ist 2-Hydroxy-4-methoxybenzophenon, bevorzugte Stabilisatoren sind 2,6-Di-tert-butyl-4-cresol und 4-Methoxyphenol.

Gegenstand der Erfindung sind auch die Ausgangsgemische zur Herstellung der Mikrokapseln für die beschriebenen Dentalmassen, welche
i) zu verkapselnde(n) Photoinitiator(en)
ii) mindestens ein polymerisierbaren Monomer
iii) mindestens eine ultrahydrophobe Verbindung und
iv) mindestens einen Initiator zur thermischen Initiierung der radikalischen Polymerisation, enthalten.

Bezüglich der Mengen- und Stoffangaben zu den Komponenten i) - iv) sei auf die obige Beschreibung verwiesen.

Die Erfindung wird im Folgenden anhand von Beispielen näher erläutert.

### Beispiele

### Beispiele zur Herstellung der mikroverkapselten Photoinitiatoren (I) und (II)

### Beispiel 1: PMMA-Verkapselung von Photoinitiator (I)

Zur Herstellung der dispersen Phase werden 1.0 g 2,4,6-Trimethylbenzoyl-diphenylphosphinoxid (Lucirin® TPO), 5.0 g Methylmethacrylat, 250 mg Hexadecan und 100 mg 2,2'-Azobis-(2-methylbutyl-1-nitrit) im 50 ml Becherglas vermischt und unter Lichtausschluss gerührt, bis das Lucirin® TPO vollständig gelöst ist. Für die kontinuierliche Phase werden 72 mg Natriumdodecylsulfat in 24.0 g demineralisiertem Wasser gelöst. Anschließend wird die kontinuierliche Phase unter Lichtausschluss und kontinuierlichem Rühren zur dispersen Phase gegeben und 1 h bei 2000 U*min⁻¹ gerührt. Nach Entfernen des Magnetrührstabs wird die entstandene Makro-Emulsion mittels eines Ultraschallstabes (½" Spitze) für eine Schalldauer von 2 min bei 90% Amplitude im Eisbad miniemulgiert. Die Probe wird in einen 50 ml Braunglaskolben überführt, fest verschlossen und über Nacht unter kontinuierlichem Rühren bei 1000 U*min⁻¹ in einem bereits auf 72°C vorgeheizten Ölbad polymerisiert. Nach Entnahme aus dem Ölbad wird die Suspension warm filtriert und gefriergetrocknet. Zur Aufreinigung und Entfernung von unverkapseltem Lucirin® TPO werden die Kapseln auf einen Büchnertrichter gegeben, mit 30 ml Ethanol versetzt und anschließend Vakuum angelegt. Durch erneute Gefriertrocknung wird restliches Ethanol entfernt, hierdurch wird eine Quellung der Kapseln verhindert. Zur Bestimmung des Lucirin® TPO Gehalts werden 60 mg des getrockneten Kapselmaterials mit 2.0 mg Pyrazin (1,4-Diazin) in 1 ml CDCl₃ gelöst und im Braunglas-NMR-Röhrchen vermessen. Im ¹H-NMR-Spektrum dient Pyrazin als Referenz zur quantitativen Bestimmung des enthaltenen Lucirin® TPO. Die mit der dynamischen Lichtstreuung (DLS) bestimmte Teilchengröße beträgt 132 nm, der Lucirinanteil in den Kapseln beträgt 28.71 Gew.% des ursprünglich eingesetzten Lucirins (entspricht ca. 5 Gew.% reinem Lucirinanteil der Kapseln).

### Beispiel 2: Copolymerverkapselung von Photoinitiator (I)

Statt Methylmethacrylat (MMA) als einziges Monomer wird eine Mischung aus Butylacrylat (BA) und Methylmethacrylat als Monomermischung eingesetzt. Die Menge beträgt 5 g. Die mit der DLS bestimmte Teilchengröße, der Luciringehalt sowie die kalorimetrisch bestimmte Glasübergangstemperatur (*T_{g}*) der Proben sind in Tabelle 1 zusammengefasst. In Tabelle 1 ist deutlich zu erkennen, dass durch die Zugabe von Butylacrylat die Glastemperatur der Polymerhülle der Partikel gesenkt wird.

**Tabelle 1: Copolymerverkapselte Lucirinproben**

| Probe | Monomer-mischung [g] | Teilchengröße [nm] | *T*_{g} [°C] | Lucirin-Gehalt* [Gew.-%] |
|---|---|---|---|---|
| 2A | 2.4 g MMA | 112 | -1.1 | 83.78 (13) |
| | 2.6 g BA | | | |
| 2B | 2.6 g MMA | 132 | 6.0 | 97.02 (15) |
| | 2.4 g BA | | | |
| 2C | 3.0 g MMA | 108 | 28.9 | 77.79 (12.5) |
| | 2.0 q BA | | | |
| 2D | 3.4 g MMA | 106 | 32.0 | 70.60 (11.5) |
| | 1.6 g BA | | | |

| | | | | |
|---|---|---|---|---|
| * erster Wert: bezogen auf eingewogene Menge Lucirin; zweiter Wert in Klammer: bezogen auf Endpartikel. | | | | |

### Beispiel 3: Molekulargewichtsgeregelte PMMA-Verkapselung von Photoinitiator (I)

Zur Herabsetzung des Molekulargewichts wird eine Mischung aus Methylmethacrylat und Butylmercaptan (BuSH) eingesetzt. Die Teilchengröße, der Luciringehalt sowie das mittels GPC bestimmte Molekulargewicht (*M*_{w}) der Proben sind in Tabelle 2 zusammengefasst

**Tabelle 2: Verkapseltes Lucirin in PMMA-Hülle mit Molekulargewichtsregler.**

| Probe | Monomer-mischung [g] | Teilchengröße [nm] | *M*_{w}[g/mol] | Lucirin-Gehalt* [Gew.-%] |
|---|---|---|---|---|
| 3A | 5.0 g MMA | 109 | 1.5·10⁵ | 55.89 (9) |
| | 6.2 mq BuSH | | | |
| 3B | 5.0 g MMA | 126 | 4.13·10⁴ | 42.00 (7) |
| | 26.8 mg BuSH | | | |
| 3C | 5.0 g MMA | 134 | 2.95·10⁴ | 68.75 (11) |
| | 48.7 mg BuSH | | | |

| | | | | |
|---|---|---|---|---|
| * erster Wert: bezogen auf eingewogene Menge Lucerin; zweiter Wert in Klammer: bezogen auf Endpartikel. | | | | |

### Beispiel 4: Vernetzte PMMA-Verkapselung von Photoinitiator (I)

Als Monomermischung wurde eine Mischung aus Methylmethacrylat und Vernetzer DBAP eingesetzt. Die Menge des Vernetzers kann in weiteren Bereichen variiert werden. Die Charakteristika sind in Tabelle 3 zusammengefasst.

**Tabelle 3: Lucirinverkapselung in vernetzter PMMA-Hülle**

| Probenbezeichnung | Monomermischung [g] | Teilchengröße [nm] | Lucirin-Gehalt* [%] |
|---|---|---|---|
| 4A | 4.5 g MMA | 107 | 89.81 (14) |
| | 0.5 g DBAP | | |
| 4B | 4.0 g MMA | 189 | 66.35(11) |
| | 1.0 g DBAP | | |

| | | | |
|---|---|---|---|
| * erster Wert: bezogen auf eingewogene Menge Lucerin; zweiter Wert in Klammer: bezogen auf Endpartikel. | | | |

### Beispiel 5: PMMA-Verkapselung von Photoinitiator (II)

### Verkapselung von Bis-(2,4,6-trimethylbenzoyl)phenylphosphinoxid (Irgacure® 819)

Zur Herstellung der dispersen Phase werden 0.7 g Irgacure® 819, 5.3 g Methylmethacrylat, 250 mg Hexadecan und 100 mg 2,2'-Azobis(2-methylbutyl-1-nitril) im 50 ml Becherglas (hohe Form) vermischt und unter Lichtausschluss gerührt, bis das Irgacure® 819 vollständig gelöst ist. Für die kontinuierliche Phase werden 72 mg Natriumdodecylsulfat (SDS) in 24.0 g demineralisiertem Wasser gelöst. Anschließend wird die kontinuierliche Phase unter Lichtausschluss und kontinuierlichem Rühren zur dispersen Phase gegeben und 1 h bei 2000 U*min⁻¹ gerührt (ebenfalls unter Lichtausschluss). Nach Entfernen des Magnetrührstabs wird die entstandene Makro-Emulsion mittels eines Ultraschallstabes (½" Spitze) für eine Schalldauer von 2 min bei 90% Amplitude im Eisbad miniemulgiert. Die Probe wird in einen 50 ml Braunglaskolben überführt, fest verschlossen und über Nacht unter kontinuierlichem Rühren bei 1000 U*min⁻¹ in einem bereits auf 72 °C vorgeheizten Ölbad polymerisiert. Nach Entnahme aus dem Ölbad wird die Suspension warm filtriert und gefriergetrocknet. Die mittels dynamischer Lichtstreuung bestimmte Teilchengröße der Partikel beträgt ca. 170 nm.

### Freisetzungskinetik des verkapselten Lucirin® TPO:

100.0 mg gefriergetrocknete, aufgereinigte Kapseln hergestellt nach Beispiel 1 bzw. 4A werden in 1.0 ml isopropanolischer Pyrazin (1,4-Diazin) -Lösung für eine definierte Zeit dispergiert und die Kapseln anschließend durch einen Spritzenfilter mit Vorfilterabfiltriert. Das Filtrat wird mit einem externen Standard aus D₂O (deuteriertes Wasser, Deuteriumoxid) im ¹H-NMR Spektrometer vermessen. Pyrazin dient hierbei als Referenz zur quantitativen Bestimmung der freigesetzten Lucirin® TPO Menge. Hierbei wird der in den Kapseln enthaltene maximale Lucirin® TPO Gehalt als 100 % Freisetzung gewertet.

Die Freisetzung des Initiators aus den nach Beispiel 1 und 4A hergestellten Kapseln wurde in 2-Propanol untersucht (Abbildung 1). Es ist zu erkennen, dass die Freisetzungszeit von 10 min für reine PMMA-Partikel auf eine Freisetzungszeit von 90 min für die vernetzten PMMA-Partikel ansteigt. Dies zeigt, dass die Freisetzungskinetik des verkapselten Initators durch Variation der Polymerhülle einfach und in weiten Grenzen beeinflusst werden kann. Eine Anpassung der Freisetzungsgeschwindigkeit der verkapselten Initiatorsysteme auf die jeweils eingesetzten Monomermischungen ist so möglich.

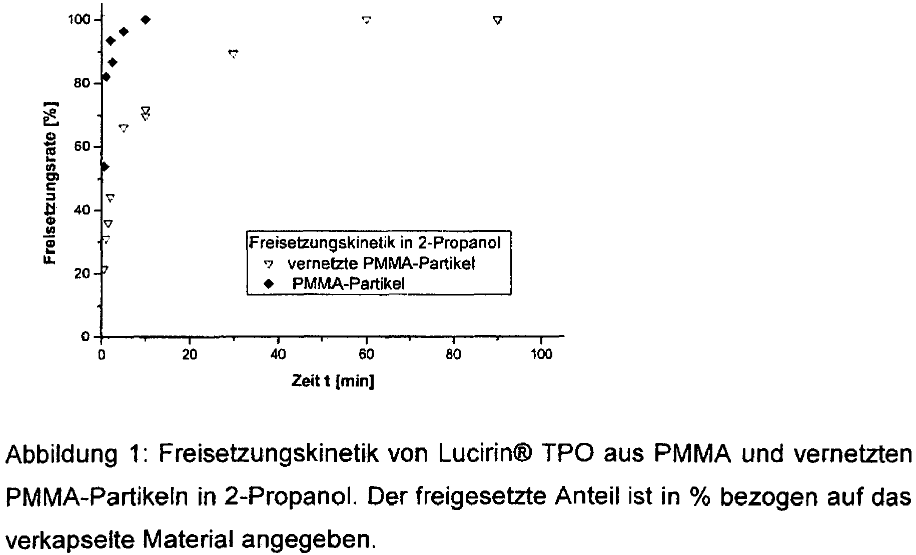

### Beispiel Herstellung mikroverkapselter Coinitiator (III)

### Beispiel 6: PMMA-Verkapselung von Amincoinitiator (III)

Zur Herstellung der dispersen Phase werden 1.0 g p-Dimethylamino-Benzoesäureethylester (EMBO), 4.5 g Methylmethacrylat, 0.5 g N,N'-diethyl-N,N'-diacrylpropylendiamin, 250 mg Hexadecan und 100 mg 2,2'-Azobis-(2-methylbutyl-1-nitril) im 50 ml Becherglas (hohe Form) vermischt und gerührt, bis das EMBO vollständig gelöst ist. Für die kontinuierliche Phase werden 72 mg Natriumdodecylsulfat in 24.0 g demineralisiertem Wasser gelöst. Anschließend wird die kontinuierliche Phase unter kontinuierlichem Rühren zur dispersen Phase gegeben und 1 h bei 2000 U*min⁻¹ gerührt. Nach Entfernen des Magnetrührstabs wird die entstandene Makro-Emulsion mittels eines Ultraschallstabes (½" Spitze) für eine Schalldauer von 2 min bei 90% Amplitude im Eisbad miniemulgiert. Die Probe wird in einen 50 ml Glaskolben überführt, fest verschlossen und über Nacht unter kontinuierlichem Rühren bei 1000 U*min⁻¹ in einem bereits auf 72 °C vorgeheizten Ölbad polymerisiert. Nach Entnahme aus dem Ölbad wird die Suspension warm filtriert und gefriergetrocknet.

Der mit der DLS bestimmte Durchmesser der Kapseln beträgt 114 nm, der Gehalt an Lucirin in den Kapseln wurde mit 15.7% bestimmt.

### Beispiele Dentalmaterialien

Die erfindungsgemäß verkapselten Initiatorsysteme wurden in folgende für Adhäsive im Dentalbereich verwendete Monomermischungen eingearbeitet:
Dentinadhäsivmischung 1:
   Zur Herstellung der Monomermischung werden 15 % 2-Hydroxyethylmethacrylat, 10 % DPEAE, 30% Bis-GMA, 20 % UDMA und 25 % Ethanol mit einem Magnetrührer vermischt und 30 min gerührt.
Dentinadhäsivmischung 2:
   Zur Herstellung der Monomermischung werden 25% Wasser, 50% DBAP, 15% BMPP und 10% AAA mit einem Magnetrührer vermischt und 30 min gerührt.

### Beispiel 7:

### Dentinadhäsiv 1, verkapselten Photoinitiator (I) enthaltend

Zur Herstellung der Adhäsivformulierung (7A) werden 99 % Dentinadhäsivmischung 1 sowie 1.0 % nach Beispiel 4A verkapseltes Lucirin® TPO mit einem Magnetrührer vermischt. Die erhaltene Lösung wird bei 42°C im Trockenschrank gelagert und nach regelmäßigen Zeitabständen die Polymerisationszeit nach ISO 6874 (1988) bestimmt. Mit Hilfe der Polymerisationszeit kann die einwandfreie Funktion des Initiators in der Adhäsivformulierung kontrolliert werden. Eine Verlangsamung der Polymerisation und damit eine Verlängerung der Polymerisationszeit ist ein eindeutiger Hinweis auf einen Abbau des Initiatorsystems innerhalb der Formulierung. Die Polymerisationszeiten sind in Tabelle 4 aufgeführt.

Als Vergleich dient eine Adhäsivformulierung (7B) bestehend aus 99,85% Dentinadhäsivmischung 2 sowie 0,15% Lucirin® TPO.

Wie aus Tabelle 4 hervorgeht, zeigt der in Formulierung 7A eingesetzte verkapselte Initiator eine im Vergleich zum nicht verkapselten System deutlich verbesserte Lagerstabilität. Durch die langsame Freisetzung des Initiators in Formulierung 7A ist zu Beginn der Lagerstabilitätsuntersuchung nur eine relativ langsame Polymerisation und damit lange Polymerisationszeit der Formulierung zu beobachten. Durch Zugabe einer definierten Menge nicht verkapselten Initiators kann dieser Effekt umgangen werden, wie dies in Beispiel 7C gezeigt ist. Zur Herstellung der Adhäsivformulierung (7C) werden 99.57 % Dentinadhäsivmischung 1, 0.33 % nach Beispiel 4A verkapseltes sowie 0.10% nicht verkapseltes Lucirin® TPO mit einem Magnetrührer vermischt. Wie in Tabelle 4 gezeigt wird, bleibt die Polymerisationszeit von Formulierung 7C während des gesamten Untersuchungszeitraums konstant.

**Tabelle 4**

| Lagerzeit (42°C) Polymerisationszeit (s) | 0 Tage | 14 Tage | 28 Tage | 56 Tage | 84 Tage | 112 Tage |
|---|---|---|---|---|---|---|
| Formulierung 7A | > 60 s | 30 s | 15s | 13s | 12s | 13s |
| Formulierung 7B | 12s | 13 s | 12s | 19s | 34 s | 50 s |
| Formulierung 7C | 15 s | 13 s | 12s | 11 s | 13 s | 12 s |

### Beispiel 8:

### Dentinadhäsiv 2, verkapselten Photoinitiator (I) enthaltend

Zur Herstellung der Adhäsivformulierung (8A) werden 98.67 % Dentinadhäsivmischung 2 sowie 1.33 % nach Beispiel 4A verkapseltes Lucirin® TPO mit einem Magnetrührer vermischt. Die erhaltene Lösung wird bei 42°C im Trockenschrank gelagert und nach regelmäßigen Zeitabständen die Polymerisationszeit bestimmt. Die Polymerisationszeiten sind in Tabelle 5 aufgeführt.

Als Vergleich dient eine Adhäsivformulierung (8B) bestehend aus 99,8% Dentinadhäsivmischung 2 sowie 0,2% Lucirin® TPO.

Wie aus Tabelle 5 hervorgeht, zeigt der in Formulierung 8A eingesetzte verkapselte Initiator eine im Vergleich zum nicht verkapselten System deutlich verbesserte Lagerstabilität. Durch die langsame Freisetzung des Initiators in Formulierung 8A ist zu Beginn der Lagerstabilitätsuntersuchung nur eine relativ langsame Polymerisation und damit lange Polymerisationszeit der Formulierung zu beobachten. Durch Zugabe einer definierten Menge nicht verkapselten Initiators kann dieser Effekt umgangen werden, wie dies in Beispiel 8C gezeigt ist. Zur Herstellung der Adhäsivformulierung (8C) werden 99.23 % Dentinadhäsivmischung 2, 0.67 % nach Beispiel 4A verkapseltes sowie 0.10% nicht verkapseltes Lucirin® TPO mit einem Magnetrührer vermischt. Wie in Tabelle 5 gezeigt wird, bleibt die Polymerisationszeit von Formulierung 8C während des gesamten Untersuchungszeitraums konstant.

**Tabelle 5**

| Lagerzeit (42°C) Polymerisationszeit (s) | 0 Tage | 14 Tage | 28 Tage | 56 Tage | 84 Tage | 112 Tage |
|---|---|---|---|---|---|---|
| Formulierung 8A | > 60 s | 57 s | 35 s | 24 s | 26 s | 25s |
| Formulierung 8B | 27 s | 25 s | 26 s | 30 s | 45 s | 58 s |
| Formulierung 8C | 28 s | 26 s | 25 s | 27 s | 23 s | 25 s |

### Beispiel 9:

### Dentinadhäsiv 1, verkapselten Amincoinitiator (III) enthaltend

Zur Herstellung der Adhäsivformulierung (9A) werden 96.47 % Dentinadhäsivmischung 1, 0,2% Campherchinon sowie 3.33 % nach Beispiel 6 verkapselter p-Dimethylamino-Benzoesäureethylester (EMBO), mit einem Magnetrührer vermischt. Die erhaltene Lösung wird bei 42°C im Trockenschrank gelagert und nach regelmäßigen Zeitabständen die Polymerisationszeit bestimmt. Die Polymerisationszeiten sind in Tabelle 6 aufgeführt.

Als Vergleich dient eine Adhäsivformulierung (9B) bestehend aus 99,3% Dentinadhäsivmischung 1, 0.2% Campherchinon sowie 0,5% p-Dimethylamino-Benzoesäureethylester (EMBO).

Wie aus Tabelle 6 hervorgeht, zeigt der in Formulierung 9A eingesetzte verkapselte Initiator eine im Vergleich zum nicht verkapselten System deutlich verbesserte Lagerstabilität. Durch die langsame Freisetzung des Initiators in Formulierung 9A ist zu Beginn der Lagerstabilitätsuntersuchung nur eine relativ langsame Polymerisation und damit lange Polymerisationszeit der Formulierung zu beobachten. Durch Zugabe einer definierten Menge nicht verkapselten Initiators kann dieser Effekt umgangen werden, wie dies in Beispiel 9C gezeigt ist. Zur Herstellung der Adhäsivformulierung (9C) werden 97.88% Dentinadhäsivmischung 1, 0,2% Campherchinon 1.67 % nach Beispiel 6 verkapseltes p-Dimethylamino-Benzoesäureethylester (EMBO) sowie 0.25% nicht verkapseltes EMBO mit einem Magnetrührer vermischt. Wie in Tabelle 6 gezeigt wird, bleibt die Polymerisationszeit von Formulierung 9C während des gesamten Untersuchungszeitraums konstant.

**Tabelle 6**

| Lagerzeit (42°C) Polymerisationszeit (s) | 0 Tage | 14 Tage | 28 Tage | 56 Tage | 84 Tage | 112 Tage |
|---|---|---|---|---|---|---|
| Formulierung 9A | 50 s | 31 s | 25 s | 27 s | 26 s | 27 s |
| Formulierung 9B | 26 s | 25s | 27 s | 35 s | 43 s | 50 s |
| Formulierung 9C | 26 s | 27 s | 24 s | 25 s | 27 s | 25 s |

### Beispiel 10:

### Dentinadhäsiv 2 verkapselten Amincoinitiator (III) enthaltend

Zur Herstellung der Adhäsivformulierung (10A) werden 96.47 % Dentinadhäsivmischung 2, 0,2% Campherchinon sowie 3.33 % nach Beispiel 6 verkapselter p-Dimethylamino-Benzoesäureethylester (EMBO), mit einem Magnet-rührer vermischt. Die erhaltene Lösung wird bei 42°C im Trockenschrank gelagert und nach regelmäßigen Zeitabständen die Polymerisationszeit bestimmt. Die Polymerisationszeiten sind in Tabelle 7 aufgeführt.

Als Vergleich dient eine Adhäsivformulierung (10B) bestehend aus 99,3% Dentinadhäsivmischung 1, 0.2% Campherchinon sowie 0,5% p-Dimethylamino-Benzoesäureethylester (EMBO).

Wie aus Tabelle 7 hervorgeht, zeigt der in Formulierung 10A eingesetzte verkapselte Initiator eine im Vergleich zum nicht verkapselten System deutlich verbesserte Lagerstabilität. Durch die langsame Freisetzung des Initiators in Formulierung 10A ist zu Beginn der Lagerstabilitätsuntersuchung nur eine relativ langsame Polymerisation und damit lange Polymerisationszeit der Formulierung zu beobachten. Durch Zugabe einer definierten Menge nicht verkapselten Initiators kann dieser Effekt umgangen werden, wie dies in Beispiel 10C gezeigt ist. Zur Herstellung der Adhäsivformulierung (10C) werden 97.88% Dentinadhäsivmischung 2, 0,2% Campherchinon, 1.67 % nach Beispiel 6 verkapseltes p-Dimethylamino-Benzoesäureethylester (EMBO) sowie 0.25% nicht verkapseltes EMBO mit einem Magnetrührer vermischt. Wie in Tabelle 7 gezeigt wird, bleibt die Polymerisationszeit von Formulierung 10C während des gesamten Untersuchungszeitraums konstant.

**Tabelle 7**

| Lagerzeit (42°C) Polymerisationszeit (s) | 0 Tage | 14 Tage | 28 Tage | 56 Tage | 84 Tage | 112 Tage |
|---|---|---|---|---|---|---|
| Formulierung 10A | > 60 s | 45 s | 42 s | 44s | 46 s | 43 s |
| Formulierung 10B | 42 s | 45 s | 46 s | 57 s | >60 s | >60 s |
| Formulierung 10C | 47 s | 44 s | 43 s | 46 s | 44s | 45 s |

## Patentansprüche

1. Mikrokapseln bestehend aus einer Hülle aus Polymeren und aus einem Kern enthaltend Photoinitiatoren, welche Acylphosphinoxide, deren Derivate oder Amincoinitiatoren oder Gemische dieser Verbindungen aufweisen wobei
a) die Photoinitiatoren Acylphosphinoxide der allgemeinen Formel (I) 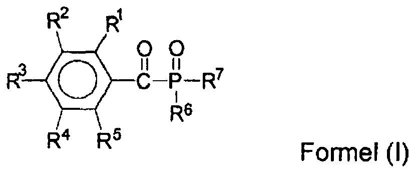 enthalten wobei
R¹, R², R³, R⁴ und R⁵ unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy substituiertes Phenyl sowie R⁶ und R⁷ 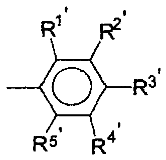 sein kann und R⁶ und R⁷ unabhängig voneinander unterschiedliche Substituenten aufweisen können, wobei R^{1'}, R^{2'}, R^{3'}, R^{4'} und R^{5'} unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxysubstituiertes Phenyl sein kann,
oder
b) die Photoinitiatoren Bisacylphosphinoxide der allgemeinen Formel (II) 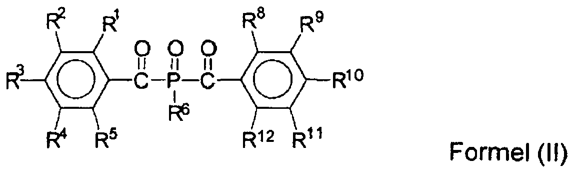 enthalten, wobei
R¹, R², R³, R⁴, R⁵, R⁶, R⁹, R¹⁰, R¹¹ und R¹² unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy-substituiertes Phenyl sein kann, sowie R⁶ 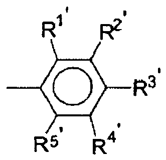 sein kann,
wobei R^{1'}, R^{2'}, R^{3'}, R^{4'}, und R^{5'} unabhängig voneinander Wasserstoff, Halogen, C₁-C₂₀-Alkyl, Cyclopentyl, Cyclohexyl, C₂-C₁₂-Alkenyl, durch ein oder mehrere O-Atome unterbrochenes C₂-C₁₈-Alkyl, durch Phenyl substituiertes C₁-C₄-Alkyl, unsubstituiertes oder mit ein oder zwei C₁-C₄-Alkyl oder/und C₁-C₄-Alkoxy substituiertes Phenyl sein kann,
oder
c) die Photoinitiatoren Amincoinitiatoren der allgemeinen Formel (III) 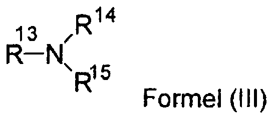 enthalten, wobei
R¹³ C₁-C₂₀-Alkyl, durch ein oder mehrere O-Atome unterbrochenes C₂-C₁₈-Alkyl, durch CN, OH substituiertes C₁-C₄-Alkyl sein kann, R¹⁴ und R¹⁵ unabhängig voneinander C₁-C₂₀-Alkyl, durch ein oder mehrere O-Atome unterbrochenes C₂-C₁₈-Alkyl, durch CN, OH substituiertes C₁-C₄-Alkyl, unsubstituiertes oder mit ein, zwei oder drei C₁-C₆-Alkyl oder/und mit carboxyalkyl-substituiertes Phenyl sein kann,
und wobei
d) für die Hülle als Polymere Polymethacrylate oder Polymethacrylamide oder Mischungen daraus eingesetzt werden.

2. Mikrokapseln nach Anspruch 1, **dadurch gekennzeichnet, dass** die Mikrokapseln hergestellt werden indem ein Zweiphasensystem bestehend aus einer Mischung aus
i) einem zu verkapselnden Photoinitiator (I), (II), (III) oder einer Mischung daraus,
ii) mindestens einem radikalisch polymerisierbaren Monomer,
iii) mindestens einer ultrahydrophobe Verbindung und
iv) mindestens einem Initiator zur thermischen Initiierung der radikalischen Polymerisation
in eine Mischung aus Wasser und mindestens einem Tensid gegeben wird und
vi) anschließend daraus mit Ultraschall eine stabile Miniemulsion gebildet wird, welche durch anschließende Erwärmung polymerisiert wird.

3. Mikrokapseln nach Anspruch 1 oder 2, **dadurch gekennzeichnet, dass** die Photoinitiatoren 2,4,6-Trimethylbenzoyl-Diphenyl-Phosphinoxide aufweisen.

4. Mikrokapseln nach Anspruch 1 oder 2, **dadurch gekennzeichnet, dass** sie Bis-(2,4,6-Trimethylbenzoyl)-Phenylphosphinoxid enthalten.

5. Verfahren zur Herstellung der Mikrokapseln nach einem der vorhergehenden Ansprüche, **dadurch gekennzeichnet, dass** ein Zweiphasensystem bestehend aus einer Mischung aus
i) einem zu verkapselnden Photoinitiator aus einem der vorhergehenden Ansprüche oder einer Mischung daraus,
ii) mindestens einem radikalisch polymerisierbaren Monomer,
iii) mindestens einer ultrahydrophoben Verbindung und
iv) mindestens einem Initiator zur thermischen Initiierung der radikalischen Polymerisation
in eine Mischung aus Wasser und mindestens einem Tensid gegeben wird und
vi) anschließend daraus eine stabile Miniemulsion gebildet wird, welche durch anschließende Erwärmung polymerisiert wird.

6. Verfahren nach Anspruch 5, **dadurch gekennzeichnet, dass** 0,1 - 40 Gew.-%, bevorzugt 0,5 - 20 Gew.-%, des zu verkapselnden Photoinitiators,
1,0 - 80 Gew.-%, bevorzugt 2,0 - 50 Gew.-%, des polymerisierbaren Monomers,
0,1 - 2 Gew.-%, bevorzugt 0,5 - 2 Gew.-%, der ultrahydrophoben Verbindung,
0,05 - 5 Gew.-%, bevorzugt 0,1 - 3 Gew.-%, eines Initiators zur thermischen Initiierung der radikalischen Polymerisation und
0,01 - 5 Gew.-%, bevorzugt 0,1 - 3 Gew.-%, eines Tensids eingesetzt werden, wobei die Angaben in Gew.-% sich auf jeweils 100 Gew.-% addieren.

7. Verfahren nach einem der Ansprüche 5 oder 6, **dadurch gekennzeichnet, dass** als Monomere Methacrylate oder Methacrylamide eingesetzt werden.

8. Verfahren nach einem der Ansprüche 5 bis 7, **dadurch gekennzeichnet, dass** als ultrahydrophobe Verbindungen aliphatische Kohlenwasserstoffe oder aromatische Kohlenwasserstoffe eingesetzt werden.

9. Verfahren nach einem der Ansprüche 5 bis 8, **dadurch gekennzeichnet, dass** als ultrahydrophobe Verbindungen aliphatische Kohlenwasserstoffe mit C₆- bis C₂₀-Alkanen eingesetzt werden.

10. Verfahren nach einem der Ansprüche 5 bis 9, **dadurch gekennzeichnet, dass** als Tenside ionische und nichtionische amphiphile Verbindungen eingesetzt werden.

11. Verwendung der Mikrokapseln nach einem der Ansprüche 1 bis 4 für selbstätzende, selbstkonditionierende Dentalmassen.

12. Verwendung der Mikrokapseln nach einem der Ansprüche 1 bis 4 für Dentin/Schmelzadhäsive.

## Claims

1. Microcapsules composed of a shell of polymers and of a core comprising photoinitiators which exhibit acylphosphine oxides, their derivatives or amine coinitiators or mixtures of these compounds, in which
a) the photoinitiators comprise acylphosphine oxides of the general formula (I) 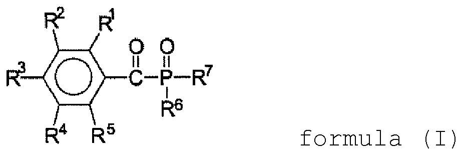 in which
R¹, R², R³, R⁴ and R⁵ can be, independently of one another, hydrogen, halogen, C₁-C₂₀-alkyl, cyclopentyl, cyclohexyl, C₂-C₁₂-alkenyl, C₂-C₁₈-alkyl interrupted by one or more oxygen atoms, C₁-C₄-alkyl substituted by phenyl, unsubstituted phenyl or phenyl substituted with one or two C₁-C₄-alkyl and/or C₁-C₄-alkoxy, and R⁶ and R⁷ can be 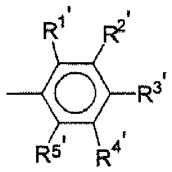 and R⁶ and R⁷ can exhibit, independently of one another, different substituents,
it being possible for R^{1'}, R^{2'}, R^{3'}, R^{4'} and R^{5'} to be, independently of one another, hydrogen, halogen, C₁-C₂₀-alkyl, cyclopentyl, cyclohexyl, C₂-C₁₂-alkenyl, C₂-C₁₈-alkyl interrupted by one or more oxygen atoms, C₁-C₄-alkyl substituted by phenyl, unsubstituted phenyl or phenyl substituted with one or two C₁-C₄-alkyl and/or C₁-C₄-alkoxy,
or
b) the photoinitiators comprise bisacylphosphine oxides of the general formula (II) 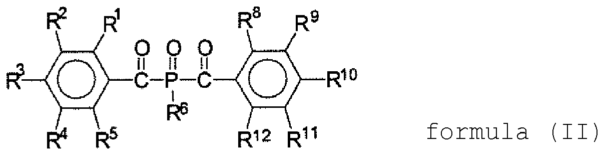 in which
R¹, R², R³, R⁴, R⁵, R⁸, R⁹, R¹⁰, R¹¹ and R¹² can be, independently of one another, hydrogen, halogen, C₁-C₂₀-alkyl, cyclopentyl, cyclohexyl, C₂-C₁₂-alkenyl, C₂-C₁₈-alkyl interrupted by one or more oxygen atoms, C₁-C₄-alkyl substituted by phenyl, unsubstituted phenyl or phenyl substituted with one or two C₁-C₄-alkyl and/or C₁-C₄-alkoxy, and R⁶ can be 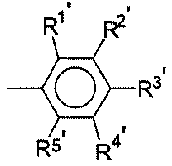 it being possible for R^{1'}, R^{2'}, R^{3'}, R^{4'} and R^{5'} to be, independently of one another, hydrogen, halogen, C₁-C₂₀-alkyl, cyclopentyl, cyclohexyl, C₂-C₁₂-alkenyl, C₂-C₁₈-alkyl interrupted by one or more oxygen atoms, C₁-C₄-alkyl substituted by phenyl, unsubstituted phenyl or phenyl substituted with one or two C₁-C₄-alkyl and/or C₁-C₄-alkoxy,
or
c) the photoinitiators comprise amine coinitiators of the general formula (III) 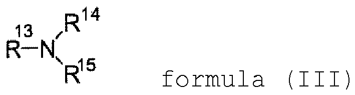 in which
R¹³ can be C₁-C₂₀-alkyl, C₂-C₁₈-alkyl interrupted by one or more oxygen atoms or C₁-C₄-alkyl substituted by CN or OH,
R¹⁴ and R¹⁵ can be, independently of one another, C₁-C₂₀-alkyl, C₂-C₁₈-alkyl interrupted by one or more oxygen atoms, C₁-C₄-alkyl substituted by CN or OH, unsubstituted phenyl or phenyl substituted with one, two or three C₁-C₆-alkyl and/or with carboxyalkyl,
and in which
d) use is made, for the shell, as polymers, of polymethacrylates or polymethacrylamides or mixtures thereof.

2. Microcapsules according to Claim 1, **characterized in that** the microcapsules are prepared by producing a two-phase system, consisting of a mixture of
i) a photoinitiator (I), (II) or (III) to be encapsulated, or a mixture thereof,
ii) at least one radically polymerizable monomer,
iii) at least one ultrahydrophobic compound and
iv) at least one initiator for the thermal initiation of the radical polymerization,
in a mixture of water and at least one surfactant, and
vi) subsequently forming therefrom, with ultrasound a stable miniemulsion, which is polymerized by subsequent heating.

3. Microcapsules according to Claim 1 or 2, **characterized in that** the photoinitiators exhibit (2,4,6-trimethylbenzoyl)diphenylphosphine oxide.

4. Microcapsules according to Claim 1 or 2, **characterized in that** they comprise bis(2,4,6-trimethylbenzoyl)phenylphosphine oxide.

5. Process for the preparation of the microcapsules according to one of the preceding claims, **characterized in that** a two-phase system, consisting of a mixture of
i) a photoinitiator to be encapsulated from one of the abovementioned claims or a mixture thereof,
ii) at least one radically polymerizable monomer,
iii) at least one ultrahydrophobic compound and
iv) at least one initiator for the thermal initiation of the radical polymerization,
in a mixture of water and at least one surfactant, is produced and
vi) subsequently a stable miniemulsion is formed therefrom and is polymerized by subsequent heating.

6. Process according to Claim 5, **characterized in that** use is made of
from 0.1 to 40% by weight, preferably from 0.5 to 20% by weight, of the photoinitiator to be encapsulated,
from 1.0 to 80% by weight, preferably from 2.0 to 50% by weight, of the polymerizable monomer,
from 0.1 to 2% by weight, preferably from 0.5 to 2% by weight, of the ultrahydrophobic compound, from 0.05 to 5% by weight, preferably from 0.1 to 3% by weight, of an initiator for the thermal initiation of the radical polymerization and
from 0.01 to 5% by weight, preferably from 0.1 to 3% by weight, of a surfactant,
the figures in % by weight adding up each time to 100% by weight.

7. Process according to either of Claims 5 and 6, **characterized in that** use is made, as monomers, of methacrylates or methacrylamides.

8. Process according to one of Claims 5 to 7, **characterized in that** use is made, as ultrahydrophobic compounds, of aliphatic hydrocarbons or aromatic hydrocarbons.

9. Process according to one of Claims 5 to 8, **characterized in that** use is made, as ultrahydrophobic compounds, of aliphatic hydrocarbons having C₆-C₂₀-alkanes.

10. Process according to one of Claims 5 to 9, **characterized in that** use is made, as surfactants, of ionic and nonionic amphiphilic compounds.

11. Use of the microcapsules according to one of Claims 1 to 4 for self-etching, self-conditioning dental materials.

12. Use of the microcapsules according to one of Claims 1 to 4 for dentin/enamel adhesives.

## Revendications

1. Microcapsules constituées d'une enveloppe à base de polymères et d'un noyau contenant des photoamorceurs qui comportent des oxydes d'acylphosphines, leurs dérivés ou des co-amorceurs amine ou des mélanges de ces composés, dans lesquelles
a) les photoamorceurs contiennent des oxydes d'acylphosphines de formule générale (I) 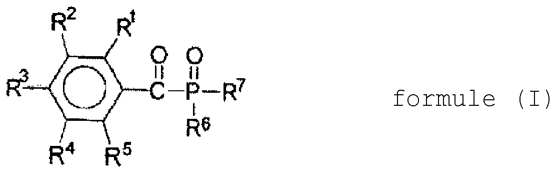 dans laquelle
R¹, R², R³, R⁴ et R⁵ peuvent être, indépendamment les uns des autres, un atome d'hydrogène ou d'halogène, un groupe alkyle en C₁-C₂₀, cyclopentyle, cyclohexyle, alcényle en C₂-C₁₂, un groupe alkyle en C₂-C₁₈ interrompu par un ou plusieurs atomes d'oxygène, un groupe alkyle en C₁-C₄ substitué par phényle, un groupe phényle non substitué ou substitué par un ou deux groupes alkyle en C₁-C₄ et/ou alcoxy en C₁-C₄, ainsi que R⁶ et R⁷ peuvent être 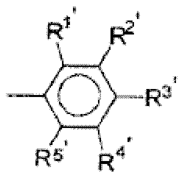 et R⁶ et R⁷ peuvent comporter, indépendamment l'un de l'autre, des substituants différents, R^{1'}, R^{2'}, R^{3'}, R^{4'} et R^{5'} pouvant être, indépendamment les uns des autres, un atome d'hydrogène ou d'halogène, un groupe alkyle en C₁-C₂₀, cyclopentyle, cyclohexyle, alcényle en C₂-C₁₂, un groupe alkyle en C₂-C₁₈ interrompu par un ou plusieurs atomes d'oxygène, un groupe alkyle en C₁-C₄ substitué par phényle, un groupe phényle non substitué ou substitué par un ou deux groupes alkyle en C₁-C₄ et/ou alcoxy en C₁-C₄,
ou
b) les photoamorceurs contiennent des oxydes de bisacylphosphines de formule générale (II) 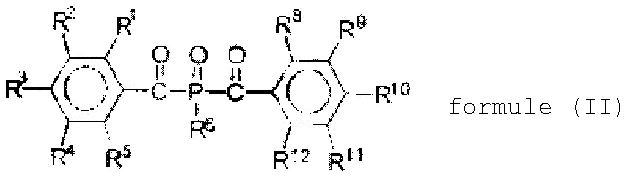 dans laquelle
R¹, R², R³, R⁴, R⁵, R⁸, R⁹, R¹⁰, R¹¹ et R peuvent être, indépendamment les uns des autres, un atome d'hydrogène ou d'halogène, un groupe alkyle en C₁-C₂₀, cyclopentyle, cyclohexyle, alcényle en C₂-C₁₂, un groupe alkyle en C₂-C₁₈ interrompu par un ou plusieurs atomes d'oxygène, un groupe alkyle en C₁-C₄ substitué par phényle, un groupe phényle non substitué ou substitué par un ou deux groupes alkyle en C₁-C₄ et/ou alcoxy en C₁-C₄, ainsi que R⁶ peut être 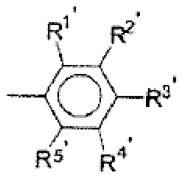 R^{1'}, R^{2'}, R^{3'}, R^{4'} et R^{5'} pouvant être, indépendamment les uns des autres, un atome d'hydrogène ou d'halogène, un groupe alkyle en C₁-C₂₀, cyclopentyle, cyclohexyle, alcényle en C₂-C₁₂, un groupe alkyle en C₂-C₁₈ interrompu par un ou plusieurs atomes d'oxygène, un groupe alkyle en C₁-C₄ substitué par phényle, un groupe phényle non substitué ou substitué par un ou deux groupes alkyle en C₁-C₄ et/ou alcoxy en C₁-C₄,
ou
c) les photoamorceurs contiennent des co-amorceurs amine de formule générale (III) 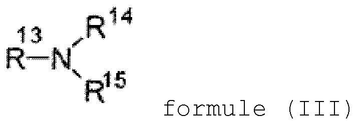
dans laquelle
R¹³ peut être un groupe alkyle en C₁-C₂₀, un groupe alkyle en C₂-C₁₈ interrompu par un ou plusieurs atomes d'oxygène, un groupe alkyle en C₁-C₄ substitué par CN, OH,
R¹⁴ et R¹⁵ peuvent être, indépendamment l'un de l'autre, un groupe alkyle en C₁-C₂₀, un groupe alkyle en C₂-C₁₈ interrompu par un ou plusieurs atomes d'oxygène, un groupe alkyle en C₁-C₄ substitué par CN, OH, un groupe phényle non substitué ou substitué par un, deux ou trois groupes alkyle en C₁-C₆ et/ou par carboxyalkyle,
et dans lesquelles
d) on utilise comme polymères pour l'enveloppe des polyméthacrylates ou des polyméthacrylamides ou des mélanges de ceux-ci.

2. Microcapsules selon la revendication 1, **caractérisées en ce qu'**on prépare les microcapsules en introduisant un système en deux phases consistant en un mélange
i) d'un photoamorceur (I), (II), (III), ou d'un mélange de tels photoamorceurs, à encapsuler,
ii) d'au moins un monomère polymérisable par voie radicalaire,
iii) d'au moins un composé ultrahydrophobe et
iv) d'au moins un amorceur pour l'amorçage thermique de la polymérisation radicalaire
dans un mélange d'eau et d'au moins un tensioactif et
vi) à la suite de cela on forme à partir de celui-ci, à l'aide d'ultrasons, une miniémulsion stable, qui est polymérisée par chauffage subséquent.

3. Microcapsules selon la revendication 1 ou 2, **caractérisées en ce que** les photoamorceurs comportent des oxydes de 2,4,6-triméthylbenzoyl-diphénylphosphines.

4. Microcapsules selon la revendication 1 ou 2, **caractérisées en ce qu'**elles contiennent de l'oxyde de bis-(2,4,6-triméthylbenzoyl)-phénylphosphine.

5. Procédé pour la préparation des microcapsules selon l'une quelconque des revendications précédentes, **caractérisé en ce qu'**on introduit un système en deux phases consistant en un mélange
i) d'un photoamorceur de l'une quelconque des revendications précédentes ou d'un mélange de tels photoamorceurs, à encapsuler,
ii) d'au moins un monomère polymérisable par voie radicalaire,
iii) d'au moins un composé ultrahydrophobe et
iv) d'au moins un amorceur pour l'amorçage thermique de la polymérisation radicalaire
dans un mélange d'eau et d'au moins un tensioactif et
vi) à la suite de cela on forme une miniémulsion stable, qui est polymérisée par chauffage subséquent.

6. Procédé selon la revendication 5, **caractérisé en ce qu'**on utilise
0,1 - 40 % en poids, de préférence 0,5 - 20 % en poids, du photoamorceur à encapsuler,
1,0 - 80 % en poids, de préférence 2,0 - 50 % en poids, du monomère polymérisable,
0,1 - 2 % en poids, de préférence 0,5 - 2 % en poids, du composé ultrahydrophobe,
0,05 - 5 % en poids, de préférence 0,1 - 3 % en poids, d'un amorceur pour l'amorçage thermique de la polymérisation radicalaire et
0,01 - 5 % en poids, de préférence 0,1 - 3 % en poids, d'un tensioactif,
la somme des données en % en poids étant chaque fois égale à 100 % en poids.

7. Procédé selon la revendication 5 ou 6, **caractérisé en ce qu'**on utilise comme monomères des méthacrylates ou des méthacrylamides.

8. Procédé selon l'une quelconque des revendications 5 à 7, **caractérisé en ce qu'**on utilise comme composés ultrahydrophobes des hydrocarbures aliphatiques ou des hydrocarbures aromatiques.

9. Procédé selon l'une quelconque des revendications 5 à 8, **caractérisé en ce qu'**on utilise comme composés ultrahydrophobes des hydrocarbures aliphatiques avec des alcanes en C₆-C₂₀.

10. Procédé selon l'une quelconque des revendications 5 à 9, **caractérisé en ce qu'**on utilise comme tensioactifs des composés amphiphiles ioniques et non ioniques.

11. Utilisation des microcapsules selon l'une quelconque des revendications 1 à 4, pour des matières dentaires automordançantes et autoconditionnantes.

12. Utilisation des microcapsules selon l'une quelconque des revendications 1 à 4, pour adhésifs pour émail/dentine.
